# EUROPEAN PATENT SPECIFICATION

(11) **EP 2 186 514 B1**
(45) Date of publication and mention of the grant of the patent: **29.06.2016**
(21) Application number: 08169136.2
(22) Date of filing: 14.11.2008
(51) Int. Cl.: A61K 31/416, A61K 31/506, A61K 45/06, A61P 35/00

(54) **Treatment of Malignant Peripheral Nerve Sheath Tumors**
Behandlung von malignen Peripherienervmantel-Tumoren
Traitement de tumeurs malignes de la gaine des nerfs périphériques

(43) Date of publication of application: 19.05.2010
(73) Proprietor: Kinki University, Higashi-Osaka-shi Osaka 577-8502 (JP)
(72) Inventor: Naoki, Sakata, Osaka 589-8511 (JP); Masatomo, Kimura, Osaka 589-8511 (JP); Mikiko, Aoki, Fukuoka 814-0180 (JP)
(74) Representative: Krauss, Jan

(56) References cited:
- WO-A-2004/005281
- WO-A-2007/065898
- WO-A-2008/116161
- KAMATH RAVINDRA ET AL: "c-Abl kinase regulates curcumin-induced cell death through activation of c-Jun N-terminal kinase" MOLECULAR PHARMACOLOGY, vol. 71, no. 1, January 2007 (2007-01), pages 61-72 URL, XP002523591 ISSN: 0026-895X
- HOLTKAMP NIKOLA ET AL: "Mutation and expression of PDGFRA and KIT in malignant peripheral nerve sheath tumors, and its implications for imatinib sensitivity" CARCINOGENESIS (OXFORD), vol. 27, no. 3, March 2006 (2006-03), pages 664-671, XP008105187 ISSN: 0143-3334
- AOKI, MIKIKO ET AL: "Imatinib mesylate inhibits cell invasion of malignant peripheral nerve sheath tumor induced by platelet-derived growth factor-BB" LABORATORY INVESTIGATION , 87(8), 767-779 CODEN: LAINAW; ISSN: 0023-6837, 2007, XP002523678
- MINGO-SION A M ET AL: "Inhibition of JNK reduces G2/M transit independent of p53, leading to endoreduplication, decreased proliferation, and apoptosis in breast cancer cells" ONCOGENE, NATURE PUBLISHING GROUP, GB BASINGSTOKE, HANTS, vol. 23, no. 2, 15 January 2004 (2004-01-15), pages 596-604, XP002408932 ISSN: 0950-9232
- O'HARE T ET AL: "AMN107: Tightening the grip of imatinib" CANCER CELL, CELL PRESS, US, vol. 7, no. 2, 1 January 2005 (2005-01-01), pages 117-119, XP008097709 ISSN: 1535-6108
- GOLEMOVIC MIRNA ET AL: "AMN107, novel aminopyrimidine inhibitor of Bcr-Abl, is significantly more potent than imatinib mesylate against Philadelphia chromosome positive acute lymphoblastic leukemia cells" BLOOD, AMERICAN SOCIETY OF HEMATOLOGY, US, vol. 104, no. 11, Part 1, 1 November 2004 (2004-11-01), page 748A, XP002365964 ISSN: 0006-4971
- KANTARJIAN HAGOP ET AL: "NILOTINIB IN IMATINIB-RESISTANT CML AND PHILADELPHIA CHROMOSOME-POSITIVE" NEW ENGLAND JOURNAL OF MEDICINE, MASSACHUSETTS MEDICAL SOCIETY, BOSTON, MA, US, vol. 354, no. 24, 15 June 2006 (2006-06-15), pages 2542-2551, XP009072571 ISSN: 1533-4406

## Description

The invention is defined by the appended set of claims.

Mutations in the NF1 tumor suppressor gene cause neurofibromatosis type 1 (NF1), a common pandemic human genetic disorder that affects approximately 250 000 patients in the US, Europe and Japan alone. NF1, also known as von Recklinghausen Disease, is characterized by a triad of café-au-lait spots (skin discolorations), cutaneous neurofibromata and iris Lisch nodules. Other features of the disorder may include skeletal dysplasia, vascular dysplasias, learning disabilities, seizures and other tumors of the neural crest origin, such as pheochromocytomas. In addition, about 10-15% of NF1 patients have low-grade astrocytomas, and less commonly, ependymoas or meningiomas.

The group of D. Wade Clapp reported in Cell 135, 437-448, October 31, 2008, the effects of Imatinib in studies in a physiologically relevant NF1 mouse model. The authors describe in the same publication the efficacy of Imatinib in a Case study of a human patient suffering from plexiform neurofibromatosis.

WO2007/065898 describes the use of the pyrimidylaminobenzamide derivatives of formula I (as further defined herein below) in the treatment of non-cancerous, benign brain tumors, especially for the curative and prophylactic treatment of neurofibromatosis (NF).

Malignant peripheral nerve sheath tumors (MPNST) are very aggressive tumors with poor prognosis. Approximately half of the MPNST occur in the setting of NF1. MPNST in NF1 patients is the major cause for reduced life expectancy with only 21% of NF1 patients surviving longer than 5 years after diagnosis of MPNST (D.G. Evans, M.E. Baser, et al, Malignant peripheral nerve sheath tumors in neurofibromatosis 1; J. Med. Genet. 2002, 39, 311-314).

The role of mutation and expression of the platelet-derived growth factor receptor type A (PDGFRA) and KIT in MPNST and its implications for Imatinib sensitivity has been discussed by N. Holtkamp, A. Okuducu, et al in Carcinogenesis 2006, 27(3), 664-671. Based upon their findings, the authors concluded that MPNST patients might benefit from treatment with Imatinib.

Jun N-terminal kinase (JNK) is a cytoplasmic serine-directed protein kinase, involved in the phosphorylation and activation of c-Jun and ATF2 and plays a significant role in metabolism, growth, cell differentiation, apoptosis, and participates as a downstream signaling component in stress responses, such as those induced by hypoxin, cold shock, and hyperosmolarity. The three isoforms of JNK, known as JNK1, 2, and 3, are encoded by 3, independent genes. JNK is activated in response to inflammation, endotoxins, and environmental stress, its activation can mediate proinflammatory gene expression, cell proliferation and apoptosis. Inhibitors of JNK are known as such and might have therapeutic utility in a number of disorders ranging from neurodegenerative disease, to metabolic disorders, inflammation, cardiovascular disease, and cancer.

The current invention is a response to the need for improved therapeutic approaches in the treatment of MPNST. It has now surprisingly been demonstrated that MPNST can be successfully treated with a combination comprising (a) at least one JNK inhibitor and (b) Imatinib or a pyrimidylaminobenzamide of formula I or, respectively, a pharmaceutically acceptable salt thereof.

In samples-obtained from a 7-year old male patient with Recklinghausen's disease, who had show some clinical response upon treatment with Imatinib, immunohistological staining revealed the presence of PDGFR, but no expression of KIT or EGFR (epidermal growth factor). Specifically, in accordance with the present invention it has now been found that Imatinib suppresses the proliferation of the MPNST cell line, YST-1, in a concentration-dependent manner. It was further found that the JNK inhibitor SP600125 also suppresses the proliferation of the YST-1 cell line in a concentration-dependent manner. Furthermore, it has been discovered that SP600125 reinforces the suppression of cell proliferation effected by Imatinib. Consequently, the present results reveal that PDGFR signaling pathways can be involved in the proliferation and progression of MPNST. In addition JNK has been discovered to be a signaling element downstream of PDGFR which can be involved in the proliferation and progression of MPNST. The obtained results suggest an additive effect in the treatment of MPNST by combining a JNK inhibitor with either Imatinib, or nilotinib, or another suitable PDGFR inhibitor.

Hence, the disclosure relates to a combination comprising (a) at least one JNK inhibitor and (b) Imatinib or a pyrimidylaminobenzamide of formula I or, respectively, a pharmaceutically acceptable salt thereof.

Furthermore, the disclosure concerns the use of a combination comprising (a) at least one JNK inhibitor and (b) Imatinib having the formula 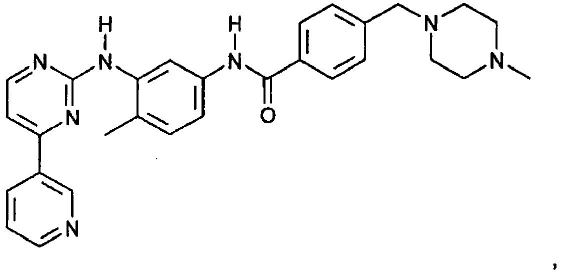 or a pyrimidylaminobenzamide of formula I 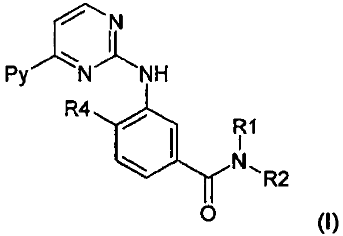 wherein
Py denotes 3-pyridyl,
R₁, represents hydrogen, lower alkyl, lower alkoxy-lower alkyl, acyloxy-lower alkyl, carboxy-lower alkyl, lower alkoxycarbonyl-lower alkyl, or phenyl-lower alkyl;
R₂ represents hydrogen, lower alkyl, optionally substituted by one or more identical or different radicals R₃, cycloalkyl, benzcycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; and
R₃ represents hydroxy, lower alkoxy, acyloxy, carboxy, lower alkoxycarbonyl, carbamoyl, N-mono- or N,N-disubstituted carbamoyl, amino, mono- or disubstituted amino, cycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted;
or wherein R₁ and R₂ together represent alkylene with four, five or six carbon atoms optionally mono- or disubstituted by lower alkyl, cycloalkyl, heterocyclyl, phenyl, hydroxy, lower alkoxy, amino, mono- or disubstituted amino, oxo, pyridyl, pyrazinyl or pyrimidinyl; benzalkylene with four or five carbon atoms; oxaalkylene with one oxygen and three or four carbon atoms; or azaalkylene with one nitrogen and three or four carbon atoms wherein nitrogen is unsubstituted or substituted by lower alkyl, phenyl-lower alkyl, lower alkoxycarbonyl-lower alkyl, carboxy-lower alkyl, carbamoyl-lower alkyl, N-mono- or N,N-disubstituted carbamoyl-lower alkyl, cycloalkyl, lower alkoxycarbonyl, carboxy, phenyl, substituted phenyl, pyridinyl, pyrimidinyl, or pyrazinyl;
R₄ represents hydrogen, lower alkyl, or halogen;
or, respectively, a pharmaceutically acceptable salt thereof for the manufacture of a pharmaceutical composition for the treatment of malignant peripheral nerve sheath tumors.

The preparation of Imatinib and the use thereof, especially as an anti-tumor agent, are described in Example 21 of European patent application EP-A-0 564 409 , which was published on 6 October 1993, and in equivalent applications and patents in numerous other countries, e.g. in US patent 5,521,184.

Pharmaceutically acceptable salts of Imatinib are pharmaceutically acceptable acid addition salts, like for example with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acrid, citric acid or oxalic acid, or amino acids such-as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid.

The monomethanesulfonic acid addition salt of Imatinib (hereinafter "Imatinib mesylate" or "Imatinib monomethanesulfonate") and a preferred crystal form thereof, especially the β-crystal form, are described in PCT patent application WO99/03854 published on January 28, 1999.

Possible pharmaceutical preparations, containing an effective amount of Imatinib or a pharmaceutically acceptable salt thereof are also described in WO99/03854.

According to the present invention, Imatinib is preferably employed in the form of the monomethanesulfonate salt, e.g. in the β-crystal form of the monomethanesulfonate salt.

Preference is also given to pyrimidylaminobenzamides of formula I, wherein py is 3-pyridyl and wherein the radicals mutually independently of each other have the following meanings:
- R₁ represents hydrogen, lower alkyl, lower alkoxy-lower alkyl, acyloxy-lower alkyl, carboxy-lower alkyl, lower alkoxycarbonyl-lower alkyl, or phenyl-lower alkyl; more preferably hydrogen;
- R₂ represents hydrogen, lower alkyl, optionally substituted by one or more identical or different radicals R₃, cycloalkyl, benzcycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted;
- R₃ represents hydroxy, lower alkoxy, acyloxy, carboxy, lower alkoxycarbonyl, carbamoyl, N-mono- or N,N-disubstituted carbamoyl, amino, mono- or disubstituted amino, cycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; and
- R₄ represents lower alkyl, especially methyl.

A preferred pyrimidylaminobenzamide is 4-methyl-3-[[4-(3-pyridinyl)-2-pyrimidinyl]amino]-*N-*[5-(4-methyl-1H-imidazol-1-yl)-3-(trifluoromethyl)phenyl] benzamide, also known as "nilotinib".

The general terms used hereinbefore and hereinafter preferably have within the context of this disclosure the following meanings, unless otherwise indicated:
The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Lower alkyl is preferably alkyl with from and including 1 up to and including 7, preferably from and including 1 to and including 4, and is linear or branched; preferably, lower alkyl is butyl, such as n-butyl, sec-butyl, isobutyl, tert-butyl, propyl, such as n-propyl or isopropyl, ethyl or methyl. Preferably lower alkyl is methyl, propyl or tert-butyl.

Lower acyl is preferably formyl or lower alkylcarbonyl, in particular acetyl.

An aryl group is an aromatic radical which is bound to the molecule via a bond located at an aromatic ring carbon atom of the radical. In a preferred embodiment, aryl is an aromatic radical having 6 to 14 carbon atoms, especially phenyl, naphthyl, tetrahydronaphthyl, fluorenyl or phenanthrenyl, and is unsubstituted or substituted by one or more, preferably up to three, especially one or two substituents, especially selected from amino, mono- or disubstituted amino, halogen, lower alkyl, substituted lower alkyl, lower alkenyl, lower alkynyl, phenyl, hydroxy, etherified or esterified hydroxy, nitro, cyano, carboxy, esterified carboxy, alkanoyl, benzoyl, carbamoyl, N-mono- or N,N-disubstituted carbamoyl, amidino, guanidino, ureido, mercapto, sulfo, lower alkylthio, phenylthio, phenyl-lower alkylthio, lower alkylphenylthio, lower alkylsulfinyl, phenylsulfinyl, phenyl-lower alkylsulfinyl, lower alkylphenylsulfinyl, lower alkylsulfonyl, phenylsulfonyl, phenyl-lower alkylsulfonyl, lower alkylphenylsulfonyl, halogen-lower alkylmercapto, halogen-lower alkylsulfonyl, such as especially trifluoromethanesulfonyl, dihydroxybora (-B(OH)₂), heterocyclyl, a mono- or bicyclic heteroaryl group and lower alkylene dioxy bound at adjacent C-atoms of the ring, such as methylene dioxy. Aryl is more, preferably phenyl, naphthyl or tetrahydronaphthyl, which in each case is either unsubstituted or independently substituted by one or two substituents selected from the group comprising halogen, especially fluorine, chlorine, or bromine; hydroxy; hydroxy etherified by lower alkyl, e.g. by methyl, by halogen-lower alkyl, e.g. trifluoromethyl, or by phenyl; lower alkylene dioxy bound to two adjacent C-atoms, e.g. methylenedioxy, lower alkyl, e.g. methyl or propyl; halogen-lower alkyl, e.g. trifluoromethyl; hydroxy-lower alkyl, e.g. hydroxymethyl or 2-hydroxy-2-propyl; lower alkoxy-lower alkyl; e.g. methoxymethyl or 2-methoxyethyl; lower alkoxycarbonyl-lower alkyl, e.g. methoxycarbonylmethyl; lower alkynyl, such as 1-propynyl; esterified carboxy, especially lower alkoxycarbonyl, e.g. methoxycarbonyl, n-propoxy carbonyl or iso-propoxy carbonyl; N-monosubstituted carbamoyl, in particular carbamoyl monosubstituted by lower alkyl, e.g. methyl, n-propyl or iso-propyl; amino; lower alkylamino, e.g. methylamino; di-lower alkylamino, e.g. dimethylamino or diefihylamino; lower alkylene-amino, e.g. pyrrolidino or piperidino; lower oxaalkylene-amino, e.g. morpholino, lower azaalkylene-amino, e.g. piperazino, acylamino, e.g. acetylamino or benzoylamino; lower alkylsulfonyl, e.g. methylsulfonyl; sulfamoyl; or phenylsulfonyl.

A cycloalkyl group is preferably cyclopropyl, cyclopentyl, cyclohexyl or cycloheptyl, and may be unsubstituted or substituted by one or more, especially one or two, substitutents selected from the group defined above as substitutents for aryl, most preferably by lower alkyl, such as methyl, lower alkoxy, such as methoxy or ethoxy, or hydroxy, and further by oxo or fused to a benzo ring, such as in benzcyclopentyl or benzcyclohexyl.

Substituted alkyl is alkyl as last defined, especially lower alkyl, preferably methyl; where one or more, especially up to three, substituents may be present, primarily from the group selected from halogen, especially fluorine, amino, N-lower alkylamino, N,N-di-lower alkylamino, N-lower alkanoylamino, hydroxy, cyano, carboxy, lower alkoxycarbonyl, and phenyl-lower alkoxycarbonyl. Trifluoromethyl is especially preferred.

Mono- or disubstituted amino is especially amino substituted by one or two radicals selected independently of one another from lower alkyl, such as methyl; hydroxy-lower alkyl, such as 2-hydroxyethyl; lower alkoxy lower alkyl, such as methoxy ethyl; phenyl-lower alkyl, such as benzyl or 2-phenylethyl; lower alkanoyl, such as acetyl; benzoyl; substituted benzoyl, wherein the phenyl radical is especially substituted by one or more, preferably one or two, substituents selected from nitro, amino, halogen, N-lower alkylamino, N,N-di-lower alkylamino, hydroxy, cyano, carboxy, lower alkoxycarbonyl, lower alkanoyl, and carbamoyl; and phenyl-lower alkoxycarbonyl, wherein the phenyl radical is unsubstituted or especially substituted by one or more, preferably one or two, substituents selected from nitro, amino, halogen, N-lower alkylamino, N,N-di-lower alkylamino, hydroxy, cyano, carboxy, lower alkoxycarbonyl, lower alkanoyl, and carbamoyl; and is preferably N-lower alkylamino, such as N-methylamino, hydroxy-lower alkylamino, such as 2-hydroxyethylamino or 2-hydroxypropyl, lower alkoxy lower alkyl, such as methoxy ethyl, phenyl-lower alkylamino, such as benzylamino, N,N-di-lower alkylamino, N-phenyl-lower alkyl-N-lower alkylamino, N,N-di-lower alkylphenylamino, lower alkanoylamino, such as acetylamino, or a substituent selected from the group comprising benzoylamino and phenyl-lower alkoxycarbonylamino, wherein the phenyl radical in each case is unsubstituted or especially substituted by nitro or amino, or also by halogen, amino, N-lower alkylamino, N,N-di-lower alkylamino, hydroxy, cyano, carboxy, lower alkoxycarbonyl, lower alkanoyl, carbamoyl or aminocarbonylamino. Disubstituted amino is also lower alkylene-amino, e.g. pyrrolidino, 2-oxopyrrolidino or piperidino; lower oxaalkylene-amino, e.g. morpholino, or lower azaalkylene-amino, e.g. piperazino or N-substituted piperazino, such as N-methylpiperazino or N-methoxycarbonylpiperazino.

Halogen is especially fluorine, chlorine, bromine, or iodine, especially fluorine, chlorine, or bromine.

Etherified hydroxy is especially C₆-C₂₀alkyloxy, such as n-decyloxy, lower alkoxy (preferred), such as methoxy, ethoxy, isopropyloxy, or tert-butyloxy, phenyl-lower alkoxy, such as benzyloxy, phenyloxy, halogen-lower alkoxy, such as trifluoromethoxy, 2,2,2-trifluoroethoxy or 1,1,2,2-tetrafluoroethoxy, or lower alkoxy which is substituted by mono- or bicyclic heteroaryl comprising one or two nitrogen atoms, preferably lower alkoxy which is substituted by imidazolyl, such as 1H-imidazol-1-yl, pyrrolyl, benzimidazolyl, such as 1-benzimidazolyl, pyridyl, especially 2-, 3- or 4-pyridyl, pyrimidinyl, especially 2-pyrimidinyl, pyrazinyl, isoquinolinyl, especially 3-isoquinolinyl, quinolinyl, indolyl or thiazolyl.

Esterified hydroxy is especially lower alkanoyloxy, benzoyloxy, lower alkoxycarbonyloxy, such as tert-butoxycarbonyloxy, or phenyl-lower alkoxycarbonyloxy, such as benzyloxycarbonyloxy.

Esterified carboxy is especially lower alkoxycarbonyl, such as tert-butoxycarbonyl, iso-propoxycarbonyl, methoxycarbonyl or ethoxycarbonyl, phenyl-lower alkoxycarbonyl, or phenyloxycarbonyl.

Alkanoyl is primarily alkylcarbonyl, especially lower alkanoyl, e.g. acetyl.

N-Mono- or N,N-disubstituted carbamoyl is especially substituted by one or two substituents independently selected from lower alkyl, phenyl-lower alkyl and hydroxy-lower alkyl, or lower alkylene, oxa-lower alkylene or aza-lower alkylene optionally substituted at the terminal nitrogen atom.

A mono- or bicyclic heteroaryl group comprising zero, one, two or three ring nitrogen atoms and zero or one oxygen atom and zero or one sulfur atom, which groups in each case are unsubstituted or mono- or polysubstituted, refers to a heterocyclic moiety that is unsaturated in the ring binding the heteroaryl radical to the rest of the molecule in formula I and is preferably a ring, where in the binding ring, but optionally also in any annealed ring, at least one carbon atom is replaced by a heteroatom selected from the group consisting of nitrogen, oxygen and sulfur; where the binding ring preferably has 5 to 12, more preferably 5 or 6 ring atoms; and which may be unsubstituted or substituted by one or more, especially one or two, substitutents selected from the group defined above as substitutents for aryl, most preferably by lower alkyl, such as methyl, lower alkoxy, such as methoxy or ethoxy, or hydroxy. Preferably the mono- or bicyclic heteroaryl group is selected from 2H-pyrrolyl, pyrrolyl, imidazolyl, benzimidazolyl, pyrazolyl, indazolyl, purinyl, pyridyl, pyrazinyl, pyrimidinyl, pyridazinyl, 4H-quinolizinyl, isoquinolyl, quinolyl, phthalazinyl, naphthyridinyl, quinoxalyl, quinazolinyl, quinnolinyl, pteridinyl, indolizinyl, 3H-indolyl, indolyl, isoindolyl, oxazolyl, isoxazolyl, thiazolyl, isothiazolyl, triazolyl, tetrazolyl, furazanyl, benzo[d]pyrazolyl, thienyl and furanyl. More preferably the mono- or bicyclic heteroaryl group is selected from the group consisting of pyrrolyl, imidazolyl, such as 1H-imidazol-1-yl, benzimidazolyl, such as 1-benzimidazolyl, indazolyl, especially 5-indazolyl, pyridyl, especially 2-, 3- or 4-pyridyl, pyrimidinyl, especially 2-pyrimidinyl, pyrazinyl, isoquinolinyl, especially 3-isoquinolinyl, quinolinyl, especially 4- or 8-quinolinyl, indolyl, especially 3-indolyl, thiazolyl, benzo[d]pyrazolyl, thienyl, and furanyl. In one preferred embodiment of the invention the pyridyl radical is substituted by hydroxy in ortho position to the nitrogen atom and hence exists at least partially in the form of the corresponding tautomer which is pyridin-(1 H)2-one. In another preferred embodiment, the pyrimidinyl radical is substituted by hydroxy both in position 2 and 4 and hence exists in several tautomeric forms, e.g. as pyrimidine-(1H, 3H)2,4-dione.

Heterocyclyl is especially a five, six or sever membered heterocyclic system with one or two heteroatoms selected from the group comprising nitrogen, oxygen, and sulfur, which may be unsaturated or wholly or partly saturated, and is unsubstituted or substituted especially by lower alkyl, such as methyl, phenyl-lower alkyl, such as benzyl, oxo, or heteroaryl, such as 2-piperazinyl; heterocyclyl is especially 2- or 3-pyrrolidinyl, 2-oxo-5-pyrrolidinyl, piperidinyl, N-benzyl-4-piperidinyl, N-lower alkyl-4-piperidinyl, N-lower alkyl-piperazinyl, morpholinyl, e.g. 2-or 3-morpholinyl, 2-oxo-1H-azepin-3-yl, 2-tetrahydrofuranyl, or 2-methyl-1,3-dioxolan-2-yl.

Pyrimidylaminobenzamides within the scope of formula I, wherein py is 3-pyridyl and the process for their manufacture are disclosed in WO 04/005281 published on January 15 2004.

Pharmaceutically acceptable salts of pyrimidylaminobenzamides of formula I, wherein py is 3-pyridyl, are especially those disclosed in WO2007/015871. In one preferred embodiment nilotinib is employed in the form of its hydrochloride monohydrate. WO2007/015870 discloses certain polymorphs of nilotinib and pharmaceutically acceptable salts thereof useful for the present invention.

The pyrimidylaminobenzamides of formula I, wherein py is 3-pyridyl, can be administered by any route including orally, parenterally, e.g., intraperitoneally, intravenously, intramuscularly, subcutaneously, intratumorally, or rectally, or enterally. Preferably, the pyrimidyl-aminobenzamides of formula I, wherein py is 3-pyridyl, is administered orally, preferably at a daily dosage of 50-2000 mg. A preferred oral daily dosage of nilotinib is 200 - 1200 mg, e.g. 800 mg, administered as a single dose or divided into multiple doses, such as twice daily dosing.

c-Jun N-terminal kinase (JNK) inhibitors useful for the present invention are disclosed in Mini Rev. Med. Chem. 2008, 8(8), 755-66, and in the references cited therein. In one preferred embodiment of the present invention, the JNK inhibitor is SP600125 having the chemical formula

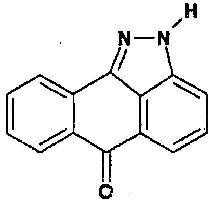

The term "treatment" as used herein means curative treatment and prophylactic treatment.

The invention pertains in particular to a pharmaceutical preparation comprising a combination as described herein for the treatment of MPNST.

Depending on species, age, individual condition, mode of administration, and the clinical picture in question, effective doses, for example daily doses of about 100-1000 mg, preferably 200-600 mg, especially 400 mg of Imatinib, are administered to warm-blooded animals of about 70 kg bodyweight. For adult patients a starting dose corresponding to 400 mg of Imatinib free base daily can be recommended. For patients with an inadequate response after an assessment of response to therapy with a dose corresponding to 400 mg of Imatinib free base daily, dose escalation can be safely considered and patients may be treated as long as they benefit from treatment and in the absence of limiting toxicities.

The disclosure provides a method for administering to a human patient having MPNST a pharmaceutically effective amount of the combination described herein to the human patient. Preferably, Imatinib or a pyrimidylaminobenzamide of formula I or a pharmaceutically acceptable salt thereof is administered once daily. The invention relates especially to such method wherein a daily dose of Imatinib mesylate corresponding to 100 to 1000 mg, e.g. 200 to 800 mg, especially 400-600 mg, preferably 400 mg, of Imatinib free base is administered.

The structure of the active agents identified by code nos., generic or trade names may be taken from the actual edition of the standard compendium "The Merck Index" or from databases, e.g. Patents International (e.g. IMS World Publications).

When the combination partners employed in the combinations as disclosed herein are applied in the form as marketed as single drugs, their dosage and mode of administration can take place in accordance with the information provided on the package insert of the respective marketed drug in order to result in the beneficial effect described herein, if not mentioned herein otherwise.

The person skilled in the pertinent art is fully enabled to select further relevant test models to prove the hereinbefore indicated beneficial effects. The pharmacological activity may, for example, be demonstrated in a clinical study.

## Claims

1. A combination comprising (a) SP600125 and (b) 4-(4-methylpiperazin-1-ylmethyl) -N-[4-methyl-3-(4-pyridin-3-yl)pyrimidin-2-ylamino)phenyl]-benzamide or 4-methyl-3-[[4-(3-pyridinyl)-2-pyrimidinyl]amino]-*N*-[5-(4-methyl-1H-imidazol-1-yl)-3-(trifluoromethyl)phenyl] benzamide, or, respectively, a pharmaceutically acceptable salt thereof, for use in the treatment of malignant peripheral nerve sheath tumors (MPNST).

2. The combination for use according to claim 1 comprising 4-(4-methylpiperazin-1 - ylmethyl)-N-[4-methyl-3-(4-pyridin-3-yl)pyrimidin-2-ylamino)phenyl]-benzamide or a pharmaceutically acceptable salt thereof for the treatment of MPNST.

3. The combination for use according to claim 2 comprising 4-(4-methylpiperazin-1 - ylmethyl)-N-[4-methyl-3-(4-pyridin-3-yl)pyrimidin-2-ylamino)phenyl]-benzamide in the form of the monomethanesulfonate salt for the treatment of MPNST.

4. The combination for use according to claim 1 comprising 4-methyl-3-[[4-(3-pyridinyl)-2-pyrimidinyl]amino]-*N*-[5-(4-methyl-1H-imidazol-1-yl)-3-(trifluoromethyl)phenyl] benzamide for the treatment of MPNST.

5. The combination for use according to claim 4 comprising 4-methyl-3-[[4-(3-pyridinyl)-2-pyrimidinyl]amino]-*N*-[5-(4-methyl-1H-imidazol-1-yl)-3-(trifluoromethyl)phenyl] benzamide in the form of its hydrochloride monohydrate for the treatment of MPNST.

6. A combination comprising (a) SP600125 and (b) 4-methyl-3-[[4-(3-pyridinyl)-2-pyrimidinyl]amino]-N-[5-(4-methyl-1 H-imidazol-1-yl)-3-(trifluoromethyl)phenyl] benzamide, or a pharmaceutically acceptable salt thereof for use in the treatment of MPNST.

## Patentansprüche

1. Eine Kombination umfassend (a) SP600125 und (b) 4-(4-Methylpiperazin-1-ylmethyl)-N-[4-methyl-3-(4-pyridin-3-yl)pyrimidin-2-ylamino)phenyl]benzamid oder 4-Methyl-3-[[4-(3-pyridinyl)-2-pyrimidinyl]amino]-N-[5-(4-methyl-1H-imidazol-1-yl)-3-(trifluoromethyl)phenyl]benzamid, oder, respektive, ein pharmazeutisch akzeptables Salz davon, zur Verwendung in der Behandlung von bösartigem Periphernervscheidentumor (MPNST).

2. Die Kombination zur Verwendung gemäß Anspruch 1 umfassend 4-(4-Methylpiperazin-1-ylmethyl)-N-[4-methyl-3-(4-pyridin-3-yl)pyrimidin-2-ylamino)phenyl]benzamid oder ein pharmazeutisch akzeptables Salz davon zur Behandlung von MPNST.

3. Die Kombination zur Verwendung gemäß Anspruch 2 umfassend 4-(4-Methylpiperazin-1-ylmethyl)-N-[4-methyl-3-(4-pyridin-3-yl)pyrimidin-2-ylamino)phenyl]benzamid in Form des Monomethansulfonat-Salzes zur Behandlung von MPNST.

4. Die Kombination zur Verwendung gemäß Anspruch 1 umfassend 4-Methyl-3-[[4-(3-pyridinyl)-2-pyrimidinyl]amino]-N-[5-(4-methyl-1H-imidazol-1-yl)-3-(trifluoromethyl)phenyl]benzamid zur Behandlung von MPNST.

5. Die Kombination zur Verwendung gemäß Anspruch 4 umfassend 4-Methyl-3-[[4-(3-pyridinyl)-2-pyrimidinyl]amino]-N-[5-(4-methyl-1H-imidazol-1-yl)-3-(trifluoromethyl)phenyl]benzamid in Form seines Hydrochloridmonohydrats zur Behandlung von MPNST.

6. Eine Zusammensetzung umfassend a) SP600125 und b) 4-Methyl-3-[[4-(3-pyridinyl)-2-pyrimidinyl]amino]-N-[5-(4-methyl-1H-imidazol-1-yl)-3-(trifluoromethyl)phenyl]benzamid, oder ein pharmazeutisch akzeptables Salz davon, zur Verwendung in der Behandlung von MPNST.

## Revendications

1. Combinaison comprenant (a) SP600125 et (b) du 4-(4-méthylpipérazin-1-ylméthyl)-N-[4-méthyl-3-(4-pyridin-3-yl)pyrimidin-2-ylamino]phényl]-benzamide ou du 4-méthyl-3-[[4-(3-pyridinyl)-2-pyrimidinyl]amino]-*N*-[5-(4-méthyl-1H-imidazol-1-yl)-3-(trifluorométhyl)phényl]benzamide, ou, respectivement, un sel pharmaceutiquement acceptable de ceux-ci, pour une utilisation dans le traitement des tumeurs malignes de la gaine des nerfs périphériques (MPNST).

2. Combinaison pour une utilisation selon la revendication 1, comprenant du 4-(4-méthylpipérazin-1-ylméthyl)-N-[4-méthyl-3-(4-pyridin-3-yl)pyrimidin-2-ylamino]phényl]-benzamide, ou un sel pharmaceutiquement acceptable de celui-ci, pour le traitement des MPNST.

3. Combinaison pour une utilisation selon la revendication 2, comprenant du 4-(4-méthylpipérazin-1-ylméthyl)-N-[4-méthyl-3-(4-pyridin-3-yl)pyrimidin-2-ylamino]phényl]-benzamide sous forme de sel monométhanesulfonate pour le traitement des MPNST.

4. Combinaison pour une utilisation selon la revendication 1, comprenant du 4-méthyl-3-[[4-(3-pyridinyl)-2-pyrimidinyl]amino]-*N*-[5-(4-méthyl-1H-imidazol-1-yl)-3-(trifluorométhyl)phényl]benzamide pour le traitement des MPNST.

5. Combinaison pour une utilisation selon la revendication 4, comprenant du 4-méthyl-3-[[4-(3-pyridinyl)-2-pyrimidinyl]amino]-*N*-[5-(4-méthyl-1H-imidazol-1-yl)-3-(trifluorométhyl)phényl]benzamide sous forme de son sel chlorhydrate monohydraté pour le traitement des MPNST.

6. Combinaison comprenant (a) SP600125 et (b) du 4-méthyl-3-[[4-(3-pyridinyl)-2-pyrimidinyl]amino]-*N*-[5-(4-méthyl-1H-imidazol-1-yl)-3-(trifluorométhyl)phényl]benzamide, ou un sel pharmaceutiquement acceptable de celui-ci, pour une utilisation dans le traitement des MPNST.
